# EUROPEAN PATENT SPECIFICATION

(11) **EP 4 346 948 B1**
(45) Date of publication and mention of the grant of the patent: **18.12.2024**
(21) Application number: 22735640.9
(22) Date of filing: 29.06.2022
(51) Int. Cl.: A61M 5/142, A61M 5/36, A61M 39/22

(54) **WEARABLE INFUSION DEVICE**
TRAGBARE INFUSIONSVORRICHTUNG
DISPOSITIF DE PERFUSION PORTABLE

(30) Priority: 05.07.2021 EP 21183717
(43) Date of publication of application: 10.04.2024
(73) Proprietor: Ivy Medical Holding B.V., 9743 AJ Groningen (NL)
(72) Inventor: TUIN, Peter Rieko, 9743 AJ Groningen (NL)
(74) Representative: V.O.
(86) International application number: PCT/NL2022/050369
(87) International publication number: WO 2023/282740

(56) References cited:
- WO-A1-2021/046082
- US-A1- 2017 188 946

## Description

The invention relates to a wearable infusion device, which is wearable on the body of a patient. More particularly, the wearable infusion device according to the invention includes at least one reservoir for holding an infusion liquid, meaning that also the at least one reservoir is to be worn on a patient's body.

Traditionally, intravenous administration of an infusion liquid to a patient is carried out by delivery from a reservoir which is suspended at a level higher than the level range of the patient body. Typically the reservoir is suspended from a pole, which is projecting from a foot on wheels, or which is mounted to a bed or to a wheelchair. Such a pole severely restricts the freedom of movement of the patient. Infusion therapy may be required for prolonged periods of time. Restricting mobility of a patient for a prolonged period of time is inconvenient for the patient, increases the need of nursery care and is particularly disadvantageous since it has been found that physical exercise and mobility has a positive effect on patient health. For these reasons, a wearable infusion device is beneficial.

However, when an infusion device including the at least one reservoir is worn on the body of an actively moving patient, the at least one reservoir and the liquid pumping mechanism of the wearable infusion device are exposed to relatively heavy movements and shocks. At times, the wearable infusion device might even be held upside-down for a while. At the same time it is important for infusion therapy that no or as little as possible air/gas bubbles are pumped into the patient.

Devices known from the prior art include for example the disclosure of WO 2021/046082 A1 and US 2017/188946 A1.

It is an object of the invention to provide at least an alternative wearable infusion device, which is able to automatically respond to relatively heavy movements and shocks in manners to the benefit of the infusion therapy that the patient is undergoing.

For that purpose the invention provides a wearable infusion device according to the appended independent claim 1. Preferable embodiments of the invention are provided by the appended dependent claims 2-9.

Hence, the invention provides a wearable infusion device, which is wearable on the body of a patient, comprising:
- at least one reservoir for holding an infusion liquid;
- a first pump, which comprises a first pump housing and a controllably driven first plunger which are bounding a first pump chamber, wherein the first plunger is reciprocally moveable to perform first intake strokes and first expel strokes, which are directed outwardly and inwardly of the first pump housing, respectively, thereby increasing and decreasing, respectively, the volume of the first pump chamber;
- a second pump, which comprises a second pump housing and a controllably driven second plunger which are bounding a second pump chamber, wherein the second plunger is reciprocally moveable to perform second intake strokes and second expel strokes, which are directed outwardly and inwardly of the second pump housing, respectively, thereby increasing and decreasing, respectively, the volume of the second pump chamber;
- a first valve, which comprises a first reservoir port, a first pump port and a first patient port; and wherein the first valve is controllably switchable between:
   - a first communication-with-the-reservoir valve position, in which the first reservoir port and the first pump port are open, and the first patient port is closed,
   - a first communication-with-the-patient valve position, in which the first pump port and the first patient port are open and the first reservoir port is closed, and
   - a first closed valve position, in which the first reservoir port, the first pump port and the first patient port are closed,
- a second valve, which comprises a second reservoir port, a second pump port and a second patient port; and wherein the second valve is controllably switchable between:
   - a second communication-with-the-reservoir valve position, in which the second reservoir port and the second pump port are open, and the second patient port is closed,
   - a second communication-with-the-patient valve position, in which the second pump port and the second patient port are open and the second reservoir port is closed, and
   - a second closed valve position, in which the second reservoir port, the second pump port and the second patient port are closed;
- a reservoir-pump fluid-path structure, which:
   - is connected to the at least one reservoir, and
   - is connected, via the first reservoir port and the first pump port of the first valve, to the first pump for transporting, in said first communication-with-the-reservoir valve position of the first valve, said infusion liquid from the at least one reservoir to the first pump, and vice versa, and
   - is connected, via the second reservoir port and the second pump port of the second valve, to the second pump for transporting, in said second communication-with-the-reservoir valve position of the second valve, said infusion liquid from the at least one reservoir to the second pump, and vice versa;
- a pump-patient fluid-path structure, which:
   - is connectable to a patient wearing the wearable infusion device, and
   - is connected, via the first patient port and the first pump port of the first valve, to the first pump for transporting, in said first communication-with-the-patient valve position of the first valve, said infusion liquid from the first pump to the patient, and vice versa, and
   - is connected, via the second patient port and the second pump port of the second valve, to the second pump for transporting, in said second communication-with-the-patient valve position of the second valve, said infusion liquid from the second pump to the patient, and vice versa;
- an orientation detector for detecting an orientation of the wearable infusion device relative to the earth's horizontal; and
- a controller configured for controlling of driving the first plunger and the second plunger, and for controlling of switching valve positions of the first valve and the second valve, and wherein the controller is further configured for determining a tilt angle alert condition in which a tilt angle detected by the orientation detector exceeds a predetermined threshold value.

Important key features of the wearable infusion device according to the invention comprise the above-mentioned at least one reservoir, first pump, second pump, first valve, second valve, orientation detector, as well as the above-mentioned controller, which is able to detect said tilt angle alert condition in which a tilt angle detected by the orientation detector exceeds a predetermined threshold value, and which is able to control the driving of the first plunger and the second plunger of the first pump and the second pump, respectively, and which is able to control the switching of the valve positions of the first valve and the second valve.

The combination of these key features allows the wearable infusion device to automatically respond to heavy movements and shocks of the wearable infusion device in many various manners to the benefit of the infusion therapy that the patient is undergoing. The following example is given to illustrate this.

Suppose that said tilt angle alert condition is detected in an operation condition of the wearable infusion device in which the first plunger of the first pump is performing a first intake stroke in the first communication-with-the-reservoir valve position of the first valve, while at the same time the second plunger of the second pump is performing a second expel stroke in the second communication-with-the-patient valve position of the second valve. In such a case, the invention allows the controller to automatically respond by pausing said first intake stroke of the first plunger, thus counter-acting that air bubbles from the at least one reservoir might enter the first pump chamber, while at the same time continuing the second expel stroke of the second plunger, thus continuing the infusion therapy. Hence, this provides the important advantage that the intake of air/gas bubbles from the at least one reservoir is prevented, while at the same time the infusion therapy is not interrupted.

To mention a further example, the invention also allows the controller to be configured to halt both the first plunger and the second plunger at some point of time during a tilt angle alert condition, if at said point of time both the first plunger and the second plunger are at maximum attainable end positions of their first and second expel strokes, respectively (which correspond to start positions of their first and second intake strokes, respectively). This prevents that air bubbles are taken into the first pump chamber and/or the second pump chamber.

It is noted that, as used herein, an orientation detector may for example be a three-axis linear accelerometer, which can derive the orientation of the detector by measuring the force gravity exerts in three mutually orthogonal (x, y, z) directions. Alternatively, as used herein, an orientation detector may for example be a gyroscope, which can measure the rotation of the detector by looking at the position of a freely rotating disk within the detector.

According to the invention, the above-mentioned predetermined threshold value for the detected tilt angle is preferably chosen in the range between 30 and 60 degrees. For example 45 degrees is a favourable value for the above-mentioned predetermined threshold value. Said tilt angle can be defined as being the angle between the earth's horizontal and a predetermined reference plane of the device, wherein said predetermined reference plane is chosen so as to be parallel to the earth's horizontal when the device is in optimal orientation with respect to preventing intake of air/gas bubbles from the at least one reservoir into the first or second pump chambers.

Furthermore it is noted that, as used herein, a bubble detector may for example be an ultrasonic bubble detector, which is able to detect gas bubbles and microbubbles inline by transmitting sound waves through a fluid path. The gas bubble can be distinguished from the liquid by looking at the velocity, attenuation and the scattering of the sound wave. Alternatively, as used herein, a bubble detector may for example be a capacitive bubble detector, which is able to detect gas bubbles of different sizes inline by looking at the capacitance between two parallel electrodes. Alternatively, as used herein, a bubble detector may for example be an optical bubble detector, which can detect air bubbles inline by transmitting light from an emitter to a collector. The intensity of the light sensed on the collector is related to whether liquid or gas passes the detector.

In a preferable embodiment of a wearable infusion device according to the invention, the controller is further configured for:
- in reaction to determining said tilt angle alert condition during a first intake stroke of the first plunger in the first communication-with-the-reservoir valve position of the first valve, pausing movement of the first plunger, while at the same time preventing the first valve to switch into the first communication-with-the-patient valve position, and resuming movement of the first plunger only after said tilt angle alert condition has ended, and
- in reaction to determining said tilt angle alert condition during a second intake stroke of the second plunger in the second communication-with-the-reservoir valve position of the second valve, pausing movement of the second plunger, while at the same time preventing the second valve to switch into the second communication-with-the-patient valve position, and resuming movement of the second plunger only after said tilt angle alert condition has ended.

In another preferable embodiment of a wearable infusion device according to the invention, the orientation detector comprises an accelerometer configured for detecting whether the wearable infusion device is in free fall and/or whether the wearable infusion device is shaking beyond a predetermined threshold-shaking level.

In a further preferable embodiment of the last-mentioned preferable embodiment of a wearable infusion device according to the invention, the controller is further configured for pausing the first pump, and/or for pausing the second pump, and/or for switching the first valve into the first closed valve position, and/or for switching the second valve into the second closed valve position, in reaction to said accelerometer having detected that the wearable infusion device is in free fall and/or that the wearable infusion device is shaking beyond said predetermined threshold-shaking level.

In another preferable embodiment of a wearable infusion device according to the invention, the orientation detector is further configured to function as a pedometer. Activity information from the pedometer can be used to assess whether the patient is actually active enough to benefit from the wearable infusion device.

In another preferable embodiment of a wearable infusion device according to the invention, the wearable infusion device further comprises:
- a reservoir-pump-path bubble detector configured and arranged for detecting bubbles in the reservoir-pump fluid-path structure,
   wherein the controller is further configured for:
   - in reaction to bubble detection by the at least one reservoir-pump-path bubble detector during a first intake stroke of the first plunger in the first communication-with-the-reservoir valve position of the first valve, preventing the first valve to switch into the first communication-with-the-patient valve position during said first intake stroke as well as during an immediately next first expel stroke of the first plunger, said immediately next first expel stroke being immediately next to said first intake stroke, and
   - in reaction to bubble detection by the at least one reservoir-pump-path bubble detector during a second intake stroke of the second plunger in the second communication-with-the-reservoir valve position of the second valve, preventing the second valve to switch into the second communication-with-the-patient valve position during said second intake stroke as well as during an immediately next second expel stroke of the second plunger, said immediately next second expel stroke being immediately next to said second intake stroke.

In a further preferable embodiment of the last-mentioned preferable embodiment of a wearable infusion device according to the invention, the controller is further configured for determining a reservoir-pump-path bubble-alert condition indicative of repeatedly detecting bubbles in the reservoir-pump fluid-path structure:
- in reaction to occurrence of bubble detection by the reservoir-pump-path bubble detector during each of a predetermined number of immediately consecutive cycles of immediately consecutive first intake strokes and first expel strokes of the first plunger in the first communication-with-the-reservoir valve position of the first valve, and/or
- in reaction to occurrence of bubble detection by the reservoir-pump-path bubble detector during each of a predetermined number of immediately consecutive cycles of immediately consecutive second intake strokes and second expel strokes of the second plunger in the second communication-with-the-reservoir valve position of the second valve.

In another preferable embodiment of a wearable infusion device according to the invention, the wearable infusion device further comprises:
- a pump-patient-path bubble detector configured and arranged for detecting bubbles in the pump-patient fluid-path structure;
   wherein the controller is further configured for determining a pump-patient-path bubble-alert condition indicative of detecting bubbles in the pump-patient fluid-path structure, in reaction to bubble detection by the pump-patient-path bubble detector in the first communication-with-the-patient valve position of the first valve, as well as in reaction to bubble detection by the pump-patient-path bubble detector in the second communication-with-the-patient valve position of the second valve.

In another preferable embodiment of a wearable infusion device according to the invention, the wearable infusion device further comprises:
- a first pump-nozzle bubble detector configured and arranged for detecting bubbles in a first nozzle of the first pump;
   wherein the controller is further configured for determining a first pump-nozzle bubble-alert condition indicative of detecting bubbles in the first nozzle of the first pump.

Said determining of said first pump-nozzle bubble-alert condition can be in reaction to bubble detection by the first pump-nozzle bubble detector in the first communication-with-the-reservoir valve position of the first valve, as well as in reaction to bubble detection by the first pump-nozzle bubble detector in the first communication-with-the-patient valve position of the first valve.

In another preferable embodiment of a wearable infusion device according to the invention, the wearable infusion device further comprises:
- a second pump-nozzle bubble detector configured and arranged for detecting bubbles in a second nozzle of the second pump;
   wherein the controller is further configured for determining a second pump-nozzle bubble-alert condition indicative of detecting bubbles in the second nozzle of the second pump.

Said determining of said second pump-nozzle bubble-alert condition can be in reaction to bubble detection by the second pump-nozzle bubble detector in the second communication-with-the-reservoir valve position of the second valve, as well as in reaction to bubble detection by the second pump-nozzle bubble detector in the second communication-with-the-patient valve position of the second valve.

In the following, the invention is further elucidated with reference to nonlimiting embodiments and with reference to the schematic figures in the appended drawing, in which the following is shown.
Fig. 1 shows, in a highly schematical way, an example of an embodiment of a wearable infusion device according to the invention.
Fig. 2 shows, in a schematical cross-sectional view, an example of an embodiment of a pump assembly of the wearable infusion device of Fig. 1.
Fig. 3 shows the situation of Fig. 2 again, however, wherein this time the pump assembly is in a different state of operation as compared to Fig. 2.
Fig. 4 shows, in the same view as that of Fig. 2, an example of an alternative embodiment of a pump assembly of the wearable infusion device of Fig. 1.

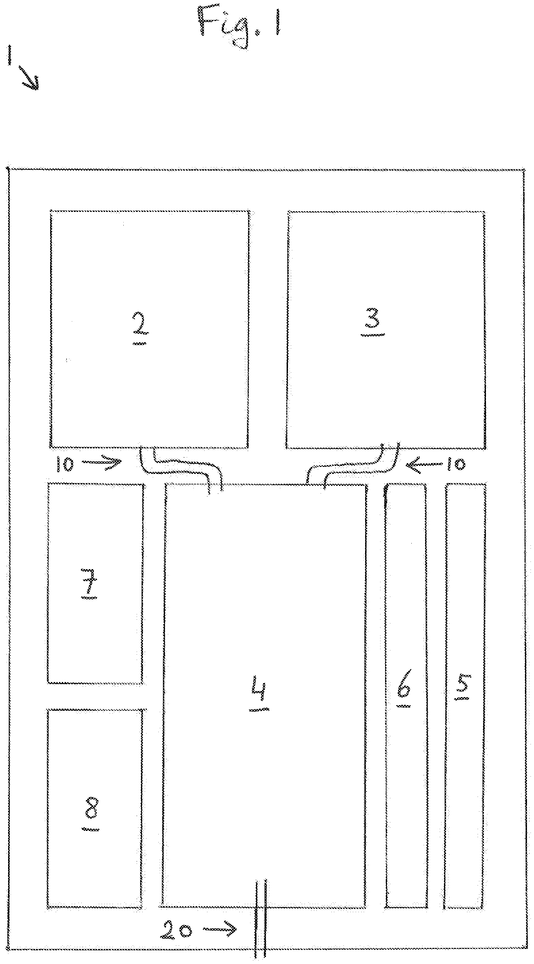

The reference signs used in Figs. 1-4 are referring to the above-mentioned parts and aspects of the invention, as well as to related parts and aspects, in the following manner.
- 1: wearable infusion device
- 2: first reservoir of said at least one reservoir
- 3: second reservoir of said at least one reservoir
- 4: pump assembly
- 5: energy source
- 6: plunger driving mechanism
- 7: orientation detector
- 8: controller
- 10: reservoir-pump fluid-path structure
- 11: first pump
- 12: second pump
- 14: reservoir-pump-path bubble detector
- 15: pump-patient-path bubble detector
- 16: first pump-nozzle bubble detector
- 17: second pump-nozzle bubble detector
- 20: pump-patient fluid-path structure
- 21: first pump housing
- 22: second pump housing
- 31: first plunger
- 32: second plunger
- 41: first pump chamber
- 42: second pump chamber
- 51: first valve
- 52: second valve
- 53: reservoir selector valve
- 61: first reservoir port (of the first valve 51)
- 62: second reservoir port (of the second valve 52)
- 63: first reservoir selection port (of the reservoir selector valve 53)
- 71: first pump port (of the first valve 51)
- 72: second pump port (of the second valve 52)
- 73: pump-side connection port (of the reservoir selector valve 53)
- 81: first patient port (of the first valve 51)
- 82: second patient port (of the second valve 52)
- 83: second reservoir selection port (of the reservoir selector valve 53)
- 91: first nozzle
- 92: second nozzle
- 104: alternative pump assembly

Based on the above introductory description, including the brief description of the drawing figures, and based on the above-listed reference signs used in Figs. 1-4, the examples of Figs. 1-4 are largly and readily self-explanatory. The following extra explanations are given.

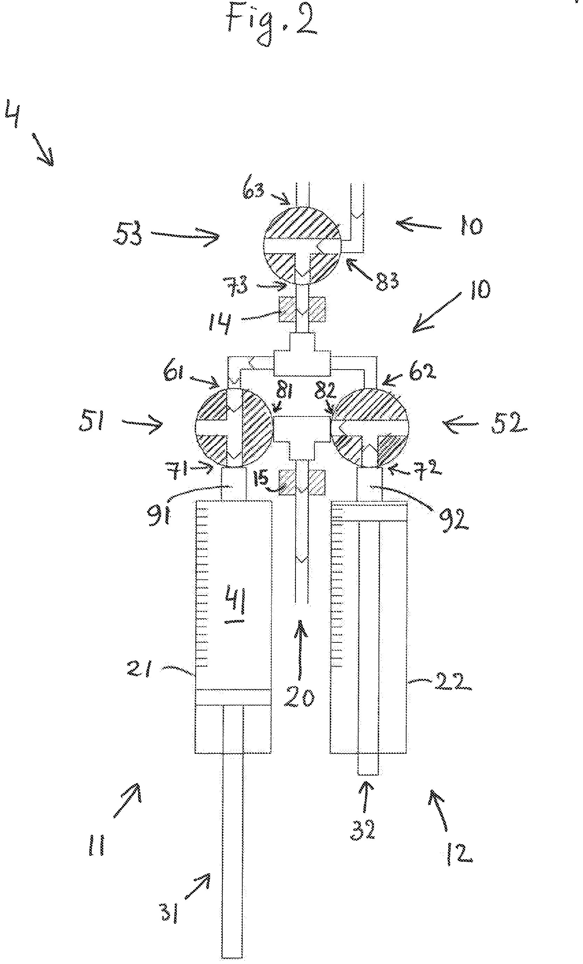

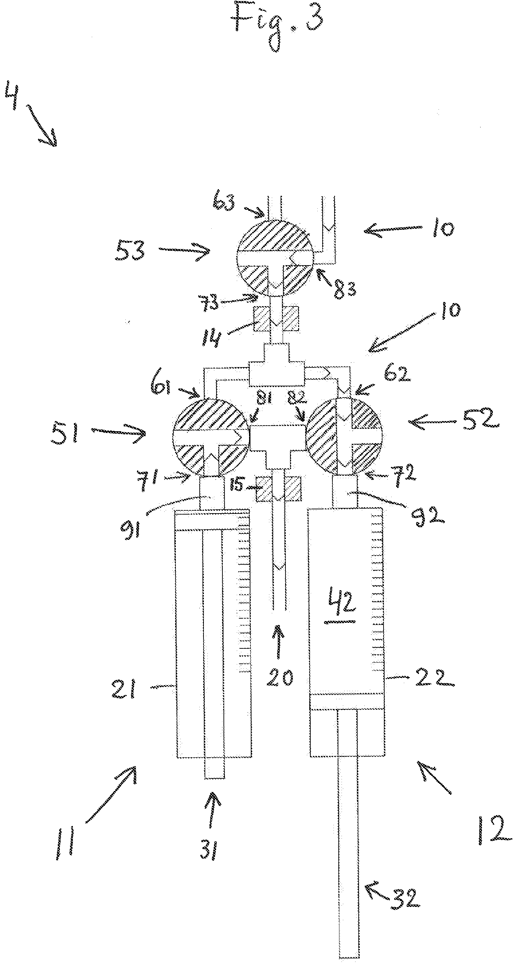

In Fig. 1 it is seen that the wearable infusion device 1 comprises the pump assembly 4. As seen in Figs. 2 and 3, the pump assembly 4 comprises at least the first pump 11, the second pump 12, the first valve 51, the second valve 52, at least part of the reservoir-pump fluid-path structure 10, at least part of the pump-patient fluid-path structure 20, the reservoir-pump-path bubble detector 14, and the the pump-patient-path bubble detector 15 of the wearable infusion device 1.

In Figs. 2 and 3 it is further seen that the pump assembly 4 further comprises the reservoir selector valve 53, which comprises the first reservoir selection port 63, the pump-side connection port 73 and the second reservoir selection port 83 for selectively connecting or disconnecting the first reservoir 2 and/or the second reservoir 3 (both shown in Fig. 1) relative to the first pump 11 and the second pump 12. For that purpose, the reservoir selector valve 53 is switchable, under control of the controller 8, between at least the following valve positions:
- the valve position which is shown in Figs. 2 and 3, and in which the first reservoir selection port 63 is closed, while the pump-side connection port 73 and the second reservoir selection port 83 are open, so that the first reservoir 2 is disconnected and the second reservoir 3 is connected (in Figs. 2 and 3 arrow signs within the reservoir selector valve 53 are indicating direction of fluid flow);
- another valve position in which the first reservoir selection port 63 and the pump-side connection port 73 are open, while the second reservoir selection port 83 is closed, so that the first reservoir 2 is connected and the second reservoir 3 is disconnected;
- yet another valve position in which the first reservoir selection port 63, the pump-side connection port 73 and the second reservoir selection port 83 are all closed, so that both the first reservoir 2 and the second reservoir 3 are disconnected.

In Fig. 1 it is further seen that the wearable infusion device 1 further comprises the energy source 5 and the plunger driving mechanism 6. The plunger driving mechanism is configured for driving the first plunger 31 and the second plunger 32. The energy source 5 (for example being an electrical battery) provides energy for various functions of the wearable infusion device 1, such as, for example, for the functions performed by the plunger driving mechanism 6, the first valve 51, the second valve 52, the reservoir selector valve 53, the controller 8, the orientation detector 7, the reservoir-pump-path bubble detector 14, the pump-patient-path bubble detector 15, the first pump-nozzle bubble detector 16 and the second pump-nozzle bubble detector 17.

Reference is now made to Fig. 2, which shows the pump assembly 4 of the wearable infusion device 1 in an operation condition, in which the first plunger 31 of the first pump 11 is performing a first intake stroke in the first communication-with-the-reservoir valve position of the first valve 51, while at the same time the second plunger 32 of the second pump 12 is performing a second expel stroke in the second communication-with-the-patient valve position of the second valve 52. In Fig. 2 arrow signs within the first valve 51, and more generally within the reservoir-pump fluid-path structure 10, are indicating direction of fluid flow from the reservoir 3 towards the first pump chamber 41 of the first pump 11. Furthermore, in Fig. 2 arrow signs within the second valve 52, and more generally within the pump-patient fluid-path structure 20, are indicating direction of fluid flow from the second pump chamber 42 (the number 42 is indicated in Fig. 3) towards the patient.

If in the operation condition of Fig. 2 the orientation detector 7 detects the tilt angle alert condition, the controller 8 automatically responds by pausing the first intake stroke of the first plunger 31, thus counter-acting that air bubbles from the reservoir 3 might enter the first pump chamber 41, while at the same time continuing the second expel stroke of the second plunger 32, thus continuing the infusion therapy. Hence, this provides the important advantage that the intake of air/gas bubbles from the at least one reservoir is prevented, while at the same time the infusion therapy is not interrupted.

Reference is now made to Fig. 3, which shows the analogous situation of Fig. 2. That is, Fig. 2 shows the pump assembly 4 of the wearable infusion device 1 in an operation condition, in which the second plunger 32 of the second pump 12 is performing a second intake stroke in the second communication-with-the-reservoir valve position of the second valve 52, while at the same time the first plunger 31 of the first pump 11 is performing a first expel stroke in the first communication-with-the-patient valve position of the first valve 51. In Fig. 3 arrow signs within the second valve 52, and more generally within the reservoir-pump fluid-path structure 10, are indicating direction of fluid flow from the reservoir 3 towards the second pump chamber 42 of the second pump 12. Furthermore, in Fig. 3 arrow signs within the first valve 51, and more generally within the pump-patient fluid-path structure 20, are indicating direction of fluid flow from the first pump chamber 41 (the number 41 is indicated in Fig. 2) towards the patient. If in the operation condition of Fig. 3 the orientation detector 7 detects the tilt angle alert condition, the controller 8 automatically responds by pausing the second intake stroke of the second plunger 32, thus counter-acting that air bubbles from the reservoir 3 might enter the second pump chamber 42, while at the same time continuing the first expel stroke of the first plunger 31, thus continuing the infusion therapy. Again, this provides the important advantage that the intake of air/gas bubbles from the at least one reservoir is prevented, while at the same time the infusion therapy is not interrupted.

It is seen that in the example of Figs. 1-3 the wearable infusion device 1 has the above-mentioned reservoir-pump-path bubble detector 14. As mentioned above the controller 8 may, in a preferable embodiment, be further configured for determining the above-mentioned reservoir-pump-path bubble-alert condition. Such a reservoir-pump-path bubble-alert condition might exist simultaneously with a tilt angle alert condition. In that case the tilt angle alert condition might have caused the reservoir-pump-path bubble-alert condition in the sense that the unfavourable orientation of the wearable infusion device 1 may have caused the detected bubbles in the reservoir-pump fluid-path structure 10. Alternatively, the wearable infusion device 1 can also be in the reservoir-pump-path bubble-alert condition, while at the same time it is not in the tilt angle alert condition. In that case an empty reservoir 2 or an empty reservoir 3 may have caused the detected bubbles in the reservoir-pump fluid-path structure 10. These examples illustrate that according to the invention the controller 8 can be configured for determining different causes of detected conditions of the wearable infusion device 1 based on combined detection results obtained via different detectors of the wearable infusion device 1.

It is further seen that in the example of Figs. 1-3 the wearable infusion device 1 further has the above-mentioned pump-patient-path bubble detector 15, with the aid of which the controller is able to determine the above-mentioned pump-patient-path bubble-alert condition.

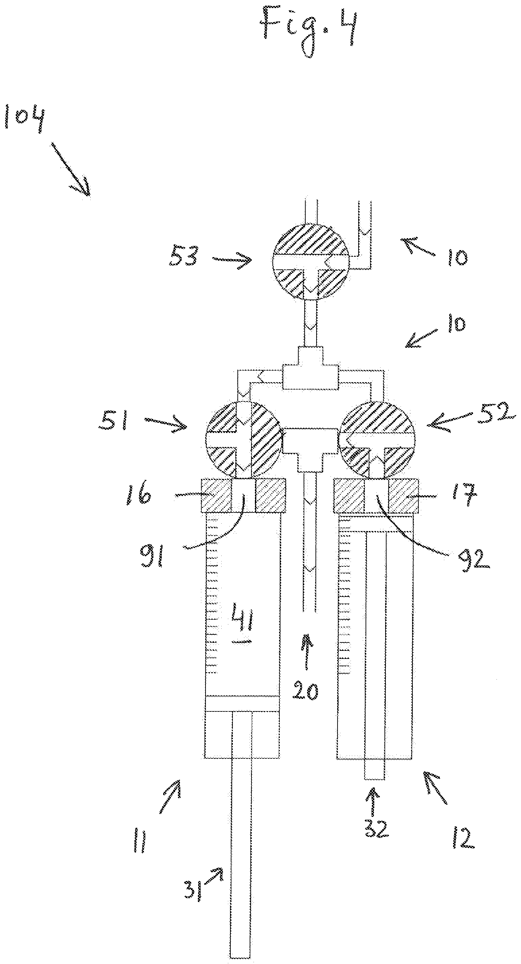

Reference is now made to Fig. 4, which shows the pump assembly 104, which is an alternative embodiment as compared to the pump assembly 4 of Figs. 2 and 3. The only difference with the pump assembly 4, is that the pump assembly 104 of Fig. 4 does not have the reservoir-pump-path bubble detector 14 and does not have the pump-patient-path bubble detector 15 of the pump assembly 4. Instead the pump assembly 104 has the above-mentioned first pump-nozzle bubble detector 16 arranged at the first nozzle 91 of the first pump 11 and the above-mentioned second pump-nozzle bubble detector 17 arranged at the second nozzle 92 of the second pump 12. With the aid of the first pump-nozzle bubble detector 16 the controller is able to determine the above-mentioned first pump-nozzle bubble-alert condition. With the aid of the second pump-nozzle bubble detector 17 the controller is able to determine the above-mentioned second pump-nozzle bubble-alert condition.

Arranging the first and second pump-nozzle bubble detectors 16 and 17 at the first nozzle 91 and the second nozzle 92, respectively, (Fig. 4) as compared to arranging the reservoir-pump-path bubble detector 14 and the pump-patient-path bubble detector 15 at the reservoir-pump fluid-path structure 10 and the pump-patient fluid-path structure 20, respectively, (Figs. 2-3) allows for a more compact arrangement of various parts of the wearable infusion device 1 relative to one another.

The invention allows the controller 8 to be configured in many various ways for controlling the first plunger 31 and/or the second plunger 32 and/or the first valve 51 and/or the second valve 52 and/or the reservoir selector valve 53 in many various ways in reaction to occurrences, in many various circumstances, of detection of the reservoir-pump-path bubble-alert condition, the pump-patient-path bubble-alert condition, the first pump-nozzle bubble-alert condition, and the second pump-nozzle bubble-alert condition.

While the invention has been described and illustrated in detail in the foregoing description and in the drawing figures, such description and illustration are to be considered exemplary and/or illustrative and not restrictive. That is, the invention is not limited to the disclosed embodiments.

For example, in the shown examples the wearable infusion device 1 has two reservoirs for holding an infusion liquid. Instead, a wearable infusion device according to the invention may also have only one such reservoir or more than two such reservoirs. Furthermore, in the shown examples the wearable infusion device 1 has the two pumps 11 and 12 and the two valves 51 and 52. Instead, a wearable infusion device according to the invention may also have more than two of such pumps and more than two of such valves. Furthermore, in the shown examples the wearable infusion device 1 either has the reservoir-pump-path bubble detector 14 and the pump-patient-path bubble detector 15, or has the first pump-nozzle bubble detector 16 and the second pump-nozzle bubble detector 17. Instead, a wearable infusion device according to the invention may also have various selections or combinations of these four detectors, such as only detector 14, only detectors 16 and 17, a combination of detectors 14, 16 and 17, a combination of detectors 14, 15, 16 and 17. Also one or more additional ones of these or other detectors may be added at various places.

## Claims

1. A wearable infusion device (1), which is wearable on the body of a patient, comprising:
• at least one reservoir (2, 3) for holding an infusion liquid;
• a first pump (11), which comprises a first pump housing (21) and a controllably driven first plunger (31) which are bounding a first pump chamber (41), wherein the first plunger is reciprocally moveable to perform first intake strokes and first expel strokes, which are directed outwardly and inwardly of the first pump housing, respectively, thereby increasing and decreasing, respectively, the volume of the first pump chamber;
• a second pump (12), which comprises a second pump housing (22) and a controllably driven second plunger (32) which are bounding a second pump chamber (42), wherein the second plunger is reciprocally moveable to perform second intake strokes and second expel strokes, which are directed outwardly and inwardly of the second pump housing, respectively, thereby increasing and decreasing, respectively, the volume of the second pump chamber;
• a first valve (51), which comprises a first reservoir port (61), a first pump port (71) and a first patient port (81); and wherein the first valve is controllably switchable between:
- a first communication-with-the-reservoir valve position, in which the first reservoir port (61) and the first pump port (71) are open, and the first patient port (81) is closed,
- a first communication-with-the-patient valve position, in which the first pump port (71) and the first patient port (81) are open and the first reservoir port (61) is closed, and
- a first closed valve position, in which the first reservoir port (61), the first pump port (71) and the first patient port (81) are closed,
• a second valve (52), which comprises a second reservoir port (62), a second pump port (72) and a second patient port (82); and wherein the second valve is controllably switchable between:
- a second communication-with-the-reservoir valve position, in which the second reservoir port (62) and the second pump port (72) are open, and the second patient port (82) is closed,
- a second communication-with-the-patient valve position, in which the second pump port (72) and the second patient port (82) are open and the second reservoir port (62) is closed, and
- a second closed valve position, in which the second reservoir port (62), the second pump port (72) and the second patient port (82) are closed;
• a reservoir-pump fluid-path structure (10), which:
- is connected to the at least one reservoir (2, 3), and
- is connected, via the first reservoir port (61) and the first pump port (71) of the first valve (51), to the first pump (11) for transporting, in said first communication-with-the-reservoir valve position of the first valve, said infusion liquid from the at least one reservoir (2, 3) to the first pump (11), and vice versa, and
- is connected, via the second reservoir port (62) and the second pump port (72) of the second valve (52), to the second pump (12) for transporting, in said second communication-with-the-reservoir valve position of the second valve, said infusion liquid from the at least one reservoir (2, 3) to the second pump (12), and vice versa;
• a pump-patient fluid-path structure (20), which:
- is connectable to a patient wearing the wearable infusion device (1), and
- is connected, via the first patient port (81) and the first pump port (71) of the first valve (51), to the first pump (11) for transporting, in said first communication-with-the-patient valve position of the first valve, said infusion liquid from the first pump (11) to the patient, and vice versa, and
- is connected, via the second patient port (82) and the second pump port (72) of the second valve (52), to the second pump (12) for transporting, in said second communication-with-the-patient valve position of the second valve, said infusion liquid from the second pump (12) to the patient, and vice versa;
• an orientation detector (7) for detecting an orientation of the wearable infusion device (1) relative to the earth's horizontal; and
• a controller (8) configured for controlling of driving the first plunger (31) and the second plunger (32), and for controlling of switching valve positions of the first valve (51) and the second valve (52), and wherein the controller (8) is further configured for determining a tilt angle alert condition in which a tilt angle detected by the orientation detector (7) exceeds a predetermined threshold value.

2. The wearable infusion device (1) according to claim 1, wherein the controller (8) is further configured for:
- in reaction to determining said tilt angle alert condition during a first intake stroke of the first plunger (31) in the first communication-with-the-reservoir valve position of the first valve (51), pausing movement of the first plunger (31), while at the same time preventing the first valve to switch into the first communication-with-the-patient valve position, and resuming movement of the first plunger (31) only after said tilt angle alert condition has ended, and
- in reaction to determining said tilt angle alert condition during a second intake stroke of the second plunger (32) in the second communication-with-the-reservoir valve position of the second valve (52), pausing movement of the second plunger (32), while at the same time preventing the second valve to switch into the second communication-with-the-patient valve position, and resuming movement of the second plunger (32) only after said tilt angle alert condition has ended.

3. The wearable infusion device (1) according to any one of the preceding claims, wherein the orientation detector (7) comprises an accelerometer configured for detecting whether the wearable infusion device is in free fall and/or whether the wearable infusion device is shaking beyond a predetermined threshold-shaking level.

4. The wearable infusion device (1) according to claim 3, wherein the controller (8) is further configured for pausing the first pump (11), and/or for pausing the second pump (12), and/or for switching the first valve (51) into the first closed valve position, and/or for switching the second valve (52) into the second closed valve position, in reaction to said accelerometer having detected that the wearable infusion device is in free fall and/or that the wearable infusion device is shaking beyond said predetermined threshold-shaking level.

5. The wearable infusion device (1) according to any one of the preceding claims, further comprising:
• a reservoir-pump-path bubble detector (14) configured and arranged for detecting bubbles in the reservoir-pump fluid-path structure (10),
and wherein the controller (8) is further configured for:
- in reaction to bubble detection by the at least one reservoir-pump-path bubble detector (14) during a first intake stroke of the first plunger (31) in the first communication-with-the-reservoir valve position of the first valve (51), preventing the first valve to switch into the first communication-with-the-patient valve position during said first intake stroke as well as during an immediately next first expel stroke of the first plunger (31), said immediately next first expel stroke being immediately next to said first intake stroke, and
- in reaction to bubble detection by the at least one reservoir-pump-path bubble detector (14) during a second intake stroke of the second plunger (32) in the second communication-with-the-reservoir valve position of the second valve (52), preventing the second valve to switch into the second communication-with-the-patient valve position during said second intake stroke as well as during an immediately next second expel stroke of the second plunger (32), said immediately next second expel stroke being immediately next to said second intake stroke.

6. The wearable infusion device (1) according to claim 5, wherein the controller (8) is further configured for determining a reservoir-pump-path bubble-alert condition indicative of repeatedly detecting bubbles in the reservoir-pump fluid-path structure (10):
- in reaction to occurrence of bubble detection by the reservoir-pump-path bubble detector (14) during each of a predetermined number of immediately consecutive cycles of immediately consecutive first intake strokes and first expel strokes of the first plunger (31) in the first communication-with-the-reservoir valve position of the first valve (51), and/or
- in reaction to occurrence of bubble detection by the reservoir-pump-path bubble detector (14) during each of a predetermined number of immediately consecutive cycles of immediately consecutive second intake strokes and second expel strokes of the second plunger (32) in the second communication-with-the-reservoir valve position of the second valve (52).

7. The wearable infusion device (1) according to any one of the preceding claims, further comprising:
• a pump-patient-path bubble detector (15) configured and arranged for detecting bubbles in the pump-patient fluid-path structure (20);
wherein the controller (8) is further configured for determining a pump-patient-path bubble-alert condition indicative of detecting bubbles in the pump-patient fluid-path structure (20), in reaction to bubble detection by the pump-patient-path bubble detector (15) in the first communication-with-the-patient valve position of the first valve (51), as well as in reaction to bubble detection by the pump-patient-path bubble detector (15) in the second communication-with-the-patient valve position of the second valve (52).

8. The wearable infusion device (1) according to any one of the preceding claims, further comprising:
• a first pump-nozzle bubble detector (16) configured and arranged for detecting bubbles in a first nozzle (91) of the first pump (11);
wherein the controller (8) is further configured for determining a first pump-nozzle bubble-alert condition indicative of detecting bubbles in the first nozzle (91) of the first pump (11).

9. The wearable infusion device (1) according to any one of the preceding claims, further comprising:
• a second pump-nozzle bubble detector (17) configured and arranged for detecting bubbles in a second nozzle (92) of the second pump (12);
wherein the controller (8) is further configured for determining a second pump-nozzle bubble-alert condition indicative of detecting bubbles in the second nozzle (92) of the second pump (12).

## Patentansprüche

1. Tragbare Infusionsvorrichtung (1), die am Körper eines Patienten tragbar ist, umfassend:
• mindestens einen Behälter (2, 3) zur Aufnahme einer Infusionsflüssigkeit;
• eine erste Pumpe (11), die ein erstes Pumpengehäuse (21) und einen steuerbar angetriebenen ersten Kolben (31) umfasst, die eine erste Pumpenkammer (41) begrenzen, wobei der erste Kolben hin- und herbewegbar ist, um erste Ansaughübe und erste Ausstoßhübe auszuführen, die nach außerhalb beziehungsweise innerhalb des ersten Pumpengehäuses gerichtet sind, wodurch das Volumen der ersten Pumpenkammer vergrößert beziehungsweise verkleinert wird;
• eine zweite Pumpe (12), die ein zweites Pumpengehäuse (22) und einen steuerbar angetriebenen zweiten Kolben (32) umfasst, die eine zweite Pumpenkammer (42) begrenzen, wobei der zweite Kolben hin- und herbewegbar ist, um zweite Ansaughübe und zweite Ausstoßhübe auszuführen, die nach außerhalb beziehungsweise innerhalb des zweiten Pumpengehäuses gerichtet sind, wodurch das Volumen der zweiten Pumpenkammer vergrößert beziehungsweise verkleinert wird;
• ein erstes Ventil (51), das einen ersten Behälteranschluss (61), einen ersten Pumpenanschluss (71) und einen ersten Patientenanschluss (81) umfasst; und wobei das erste Ventil steuerbar schaltbar ist zwischen:
- einer ersten Ventilstellung für den Austausch mit dem Behälter, in welcher der erste Behälteranschluss (61) und der erste Pumpenanschluss (71) offen sind und der erste Patientenanschluss (81) geschlossen ist,
- einer ersten Ventilstellung für den Austausch mit dem Patienten, in welcher der erste Pumpenanschluss (71) und der erste Patientenanschluss (81) offen sind und der erste Behälteranschluss (61) geschlossen ist, und
- einer ersten geschlossenen Ventilstellung, in welcher der erste Behälteranschluss (61), der erste Pumpenanschluss (71) und der erste Patientenanschluss (81) geschlossen sind,
• ein zweites Ventil (52), das einen zweiten Behälteranschluss (62), einen zweiten Pumpenanschluss (72) und einen zweiten Patientenanschluss (82) umfasst; und wobei das zweite Ventil steuerbar schaltbar ist zwischen:
- einer zweiten Ventilstellung für den Austausch mit dem Behälter, in welcher der zweite Behälteranschluss (62) und der zweite Pumpenanschluss (72) offen sind und der zweite Patientenanschluss (82) geschlossen ist,
- einer zweiten Ventilstellung für den Austausch mit dem Patienten, in welcher der zweite Pumpenanschluss (72) und der zweite Patientenanschluss (82) offen sind und der zweite Behälteranschluss (62) geschlossen ist, und
- einer zweiten geschlossenen Ventilstellung, in welcher der zweite Behälteranschluss (62), der zweite Pumpenanschluss (72) und der zweite Patientenanschluss (82) geschlossen sind;
• eine Behälter-Pumpen-Fluidbahnstruktur (10), die:
- mit dem mindestens einen Behälter (2, 3) verbunden ist, und
- über den ersten Behälteranschluss (61) und den ersten Pumpenanschluss (71) des ersten Ventils (51) mit der ersten Pumpe (11) zum Transportieren, in der Ventilstellung des ersten Ventils für den Austausch mit dem Behälter, der Infusionsflüssigkeit von dem mindestens einen Behälter (2, 3) zur ersten Pumpe (11) und umgekehrt verbunden ist, und
- über den zweiten Behälteranschluss (62) und den zweiten Pumpenanschluss (72) des zweiten Ventils (52) mit der zweiten Pumpe (12) zum Transportieren, in der Ventilstellung des zweiten Ventils für den Austausch mit dem Behälter, der Infusionsflüssigkeit von dem mindestens einen Behälter (2, 3) zur zweiten Pumpe (12) und umgekehrt verbunden ist;
• eine Pumpe-Patient-Fluidbahnstruktur (20), die:
- an einen Patienten, der die tragbare Infusionsvorrichtung (1) trägt, anschließbar ist, und
- über den ersten Patientenanschluss (81) und den ersten Pumpenanschluss (71) des ersten Ventils (51) mit der ersten Pumpe (11) zum Transportieren, in der ersten Ventilstellung des ersten Ventils für den Austausch mit dem Patienten, der Infusionsflüssigkeit von der ersten Pumpe (11) zum Patienten und umgekehrt verbunden ist, und
- über den zweiten Patientenanschluss (82) und den zweiten Pumpenanschluss (72) des zweiten Ventils (52) mit der zweiten Pumpe (12) zum Transportieren, in der zweiten Ventilstellung des zweiten Ventils für den Austausch mit dem Patienten, der Infusionsflüssigkeit von der zweiten Pumpe (12) zu dem Patienten und umgekehrt verbunden ist;
• einen Ausrichtungsdetektor (7) zum Erkennen einer Ausrichtung der tragbaren Infusionsvorrichtung (1) in Bezug auf die Erdhorizontale; und
• eine Steuerung (8), konfiguriert zum Steuern des Antreibens des ersten Kolbens (31) und des zweiten Kolbens (32) und zum Steuern des Schaltens von Ventilstellungen des ersten Ventils (51) und des zweiten Ventils (52), und wobei die Steuerung (8) ferner zum Bestimmen einer Neigungswinkelwarnbedingung konfiguriert ist, bei der ein vom Ausrichtungsdetektor (7) erfasster Neigungswinkel einen vorbestimmten Schwellenwert überschreitet.

2. Tragbare Infusionsvorrichtung (1) nach Anspruch 1, wobei die Steuerung (8) ferner dazu konfiguriert ist:
- in Reaktion auf das Feststellen der Neigungswinkelwarnbedingung während eines ersten Ansaughubs des ersten Kolbens (31) in der ersten Ventilstellung des ersten Ventils (51) für den Austausch mit dem Behälter das Bewegen des ersten Kolbens (31) zu unterbrechen, während gleichzeitig verhindert wird, dass das erste Ventil in die erste Ventilstellung für den Austausch mit dem Patienten schaltet, und die Bewegung des ersten Kolbens (31) erst nach Beendigung der Neigungswinkelwarnbedingung wieder aufzunehmen, und
- in Reaktion auf das Feststellen der Neigungswinkelwarnbedingung während eines zweiten Ansaughubs des zweiten Kolbens (32) in der zweiten Ventilstellung des zweiten Ventils (52) für den Austausch mit dem Behälter das Bewegen des zweiten Kolbens (32) zu unterbrechen, während gleichzeitig verhindert wird, dass das zweite Ventil in die zweite Ventilstellung für den Austausch mit dem Patienten schaltet, und die Bewegung des zweiten Kolbens (32) erst nach Beendigung der Neigungswinkelwarnbedingung wieder aufzunehmen.

3. Tragbare Infusionsvorrichtung (1) nach einem der vorstehenden Ansprüche, wobei der Ausrichtungsdetektor (7) einen Beschleunigungsmesser umfasst, der dazu konfiguriert ist, zu erkennen, ob sich die tragbare Infusionsvorrichtung im freien Fall befindet und/oder ob die tragbare Infusionsvorrichtung über einen vorbestimmten Schwellenwert für das Schütteln hinaus geschüttelt wird.

4. Tragbare Infusionsvorrichtung (1) nach Anspruch 3, wobei die Steuerung (8) ferner dazu konfiguriert ist, die erste Pumpe (11) anzuhalten und/oder die zweite Pumpe (12) anzuhalten, und/oder das erste Ventil (51) in die erste geschlossene Ventilstellung zu schalten und/oder das zweite Ventil (52) in die zweite geschlossene Ventilstellung zu schalten, in Reaktion darauf, dass der Beschleunigungsmesser festgestellt hat, dass sich die tragbare Infusionsvorrichtung im freien Fall befindet und/oder dass die tragbare Infusionsvorrichtung über den vorbestimmten Schwellenwert für das Schütteln hinaus geschüttelt wird.

5. Tragbare Infusionsvorrichtung (1) nach einem der vorstehenden Ansprüche, ferner umfassend:
• einen Behälter-Pumpe-Bahn-Blasendetektor (14), konfiguriert und angeordnet zum Erkennen von Blasen in der Behälter-Pumpe-Fluidbahnstruktur (10), und wobei die Steuerung (8) ferner dazu konfiguriert ist:
- in Reaktion auf Blasenerkennung durch den mindestens einen Behälter-Pumpe-Bahn-Blasendetektor (14) während eines ersten Ansaughubs des ersten Kolbens (31) in der ersten Ventilstellung des ersten Ventils (51) für den Austausch mit dem Behälter sowie während eines unmittelbar nächsten ersten Ausstoßhubs des ersten Kolbens (31), wobei der unmittelbar nächste erste Ausstoßhub unmittelbar auf den ersten Ansaughub folgt, zu verhindern, dass das erste Ventil in die erste Ventilstellung für den Austausch mit dem Patienten schaltet, und
- in Reaktion auf Blasenerkennung durch den mindestens einen Behälter-Pumpe-Bahn-Blasendetektor (14) während eines zweiten Ansaughubs des zweiten Kolbens (32) in der zweiten Ventilstellung des zweiten Ventils (52) für den Austausch mit dem Behälter sowie während eines unmittelbar nächsten zweiten Ausstoßhubs des zweiten Kolbens (32), wobei der unmittelbar nächste zweite Ausstoßhub unmittelbar auf den zweiten Ansaughub folgt, zu verhindern, dass das zweite Ventil in die zweite Ventilstellung für den Austausch mit dem Patienten schaltet.

6. Tragbare Infusionsvorrichtung (1) nach Anspruch 5, wobei die Steuerung (8) ferner dazu konfiguriert ist, einen Behälter-Pumpe-Bahnblasenwarnzustand zu bestimmen, der darauf hinweist, dass wiederholt Blasen in der Behälter-Pumpe-Fluidbahnstruktur (10) erkannt werden:
- in Reaktion auf das Auftreten von Blasenerkennung durch den Behälter-Pumpe-Bahn-Blasendetektor (14) während jedes einer vorbestimmten Anzahl von unmittelbar aufeinanderfolgenden Zyklen unmittelbar aufeinanderfolgender erster Ansaughübe und erster Ausstoßhübe des ersten Kolbens (31) in der ersten Stellung des ersten Ventils (51) für den Austausch mit dem Behälter, und/oder
- in Reaktion auf das Auftreten einer Blasenerkennung durch den Behälter-Pumpe-Bahn-Blasendetektor (14) während jedes einer vorbestimmten Anzahl von unmittelbar aufeinanderfolgenden Zyklen unmittelbar aufeinanderfolgender zweiter Ansaughübe und zweiter Ausstoßhübe des zweiten Kolbens (32) in der zweiten Stellung des zweiten Ventils (52) für den Austausch mit dem Behälter.

7. Tragbare Infusionsvorrichtung (1) nach einem der vorstehenden Ansprüche, ferner umfassend:
• einen Pumpe-Patient-Bahn-Blasendetektor (15), konfiguriert und angeordnet zum Erkennen von Blasen in der Pumpe-Patient-Fluidbahnstruktur (20);
wobei die Steuerung (8) ferner dazu konfiguriert ist, einen Pumpe-Patient-Bahn-Blasenwarnzustand festzustellen, der darauf hinweist, dass Blasen in der Pumpe-Patient-Fluidbahnstruktur (20) erkannt werden, in Reaktion auf Blasenerkennung durch den Pumpe-Patient-Bahn-Blasendetektor (15) in der ersten Ventilstellung des ersten Ventils (51) für den Austausch mit dem Patienten sowie in Reaktion auf Blasenerkennung durch den Pumpe-Patient-Bahn-Blasendetektor (15) in der zweiten Ventilstellung des zweiten Ventils (52) für den Austausch mit dem Patienten.

8. Tragbare Infusionsvorrichtung (1) nach einem der vorstehenden Ansprüche, ferner umfassend:
• einen ersten Pumpe-Düse-Blasendetektor (16), konfiguriert und angeordnet zum Erkennen von Blasen in einer ersten Düse (91) der ersten Pumpe (11);
wobei die Steuerung (8) ferner dazu konfiguriert ist, einen ersten Pumpe-Düse-Blasenwarnzustand festzustellen, der darauf hinweist, dass Blasen in der ersten Düse (91) der ersten Pumpe (11) erkannt werden.

9. Tragbare Infusionsvorrichtung (1) nach einem der vorstehenden Ansprüche, ferner umfassend:
• einen zweiten Pumpe-Düse-Blasendetektor (17), konfiguriert und angeordnet zum Erkennen von Blasen in einer zweiten Düse (92) der zweiten Pumpe (12);
wobei die Steuerung (8) ferner dazu konfiguriert ist, einen zweiten Pumpe-Düse-Blasenwarnzustand festzustellen, der darauf hinweist, dass Blasen in der zweiten Düse (92) der zweiten Pumpe (12) erkannt werden.

## Revendications

1. Dispositif de perfusion portable (1), qui peut être porté sur le corps d'un patient, comprenant :
• au moins un réservoir (2, 3) pour contenir un liquide de perfusion;
• une première pompe (11), qui comprend un logement de première pompe (21) et un premier piston entraîné de manière contrôlable (31) qui délimitent une chambre de première pompe (41), dans lequel le premier piston est mobile en va-et-vient pour effectuer des premiers courses d'admission et des premières courses d'expulsion, qui sont dirigées vers l'extérieur et vers l'intérieur du logement de première pompe, respectivement, augmentant et diminuant, respectivement, le volume de la chambre de première pompe;
• une deuxième pompe (12), qui comprend un logement de deuxième pompe(22) et un deuxième piston entraîné de manière contrôlable (32) qui délimitent une chambre de deuxième pompe (42), dans lequel le deuxième piston est mobile en va-et-vient pour effectuer des deuxièmes courses d'admission et des deuxièmes courses d'expulsion, qui sont dirigées vers l'extérieur et vers l'intérieur du logement de deuxième pompe, respectivement, augmentant et diminuant, respectivement, le volume de la chambre de deuxième pompe;
• une première valve (51), qui comprend un premier orifice de réservoir (61), un premier orifice de pompe (71) et un premier orifice de patient (81); et dans lequel la première valve peut être commutée de manière contrôlée entre :
- une première position de valve de communication avec le réservoir, dans laquelle le premier orifice de réservoir (61) et le premier orifice de pompe (71) sont ouverts, et le premier orifice de patient (81) est fermé,
- une première position de communication avec le patient, dans laquelle le premier orifice de pompe (71) et le premier orifice de patient (81) sont ouverts et le premier orifice de réservoir (61) est fermé, et
- une première position de valve fermée, dans laquelle le premier orifice de réservoir (61), le premier orifice de pompe (71) et le premier orifice de patient (81) sont fermés,
• une deuxième valve (52), qui comprend un deuxième orifice de réservoir (62), un deuxième orifice de pompe (72) et un deuxième orifice de patient (82); et dans lequel la deuxième valve peut être commutée de manière contrôlée entre:
- une deuxième position de valve de communication avec le réservoir, dans laquelle le deuxième orifice de réservoir (62) et le deuxième orifice de pompe (72) sont ouverts, et le deuxième orifice de patient (82) est fermé,
- une deuxième position de valve de communication avec le patient, dans laquelle le deuxième orifice de pompe (72) et le deuxième orifice de patient (82) sont ouverts et le deuxième orifice de réservoir (62) est fermé, et
- une deuxième position de valve fermée, dans laquelle le deuxième orifice de réservoir (62), le deuxième orifice de pompe (72) et le deuxième orifice de patient (82) sont fermés;
• une structure de trajet de fluide de pompe au réservoir (10), qui :
- est relié à l'au moins un réservoir (2, 3), et
- est raccordé, via le premier orifice de réservoir (61) et le premier orifice de pompe (71) de la première valve (51), à la première pompe (11) pour transporter, dans ladite première position de valve de communication avec le réservoir de la première valve, ledit liquide d'infusion de l'au moins un réservoir (2, 3) à la première pompe (11), et vice versa, et
- est connecté, via le deuxième orifice de réservoir (62) et le deuxième orifice de pompe (72) de la deuxième valve (52), à la deuxième pompe (12) pour transporter, dans ladite deuxième position de valve de communication avec le réservoir de la deuxième valve, ledit liquide d'infusion de l'au moins un réservoir (2, 3) à la deuxième pompe (12), et vice versa ;
• une structure de trajet de fluide de patient-pompe (20), qui :
- peut être connecté à un patient portant le dispositif de perfusion portable (1), et
- est relié, via le premier orifice de patient (81) et le premier orifice de pompe (71) de la première valve (51), à la première pompe (11) pour transporter, dans ladite première position de valve de communication avec le patient de la première valve, ledit liquide d'infusion de la première pompe (11) au patient, et vice versa, et
- est reliée, via le deuxième orifice de patient (82) et le deuxième orifice de pompe (72) de la deuxième valve (52), à la deuxième pompe (12) pour transporter, dans ladite deuxième position de valve de communication avec le patient de la deuxième valve, ledit liquide de perfusion de la deuxième pompe (12) au patient, et vice versa ;
• un détecteur d'orientation (7) pour détecter une orientation du dispositif de perfusion portable (1) par rapport à l'horizontale de la terre; et
• un dispositif de commande (8) configuré pour commander l'entraînement du premier piston plongeur (31) et du deuxième piston plongeur (32), et pour commander les positions de valve de commutation de la première valve (51) et de la deuxième valve (52), et dans lequel le dispositif de commande (8) est en outre configuré pour déterminer une condition d'alerte d'angle d'inclinaison dans laquelle un angle d'inclinaison détecté par le détecteur d'orientation (7) dépasse une valeur seuil prédéterminée.

2. Dispositif de perfusion portable (1) selon la revendication 1, dans lequel le dispositif de commande (8) est en outre configuré pour :
- en réaction à la détermination de ladite condition d'alerte d'angle d'inclinaison pendant une première course d'admission du premier piston plongeur (31) dans la première position de valve de communication avec le réservoir de la première valve (51), la pause du mouvement du premier piston plongeur (31), tout en empêchant en même temps la première valve de commuter dans la première position de valve de communication avec le patient, et de reprendre le mouvement du premier piston plongeur (31) uniquement après la fin de ladite condition d'alerte d'angle d'inclinaison, et
- en réaction à la détermination de ladite condition d'alerte d'angle d'inclinaison pendant une deuxième course d'admission du deuxième piston plongeur (32) dans la deuxième position de valve de communication avec le réservoir de la deuxième valve (52), la mise en pause du mouvement du deuxième piston plongeur (32), tout en empêchant en même temps la deuxième valve de commuter dans la deuxième position de valve de communication avec le patient, et reprendre le mouvement du deuxième piston plongeur (32) uniquement après la fin de ladite condition d'alerte d'angle d'inclinaison.

3. Dispositif de perfusion portable (1) selon l'une quelconque des revendications précédentes, dans lequel le détecteur d'orientation (7) comprend un accéléromètre configuré pour détecter si le dispositif de perfusion portable est en chute libre et/ou si le dispositif portable le dispositif de perfusion est secoué au-delà d'un niveau de tremblement de seuil prédéterminé.

4. Dispositif de perfusion portable (1) selon la revendication 3, dans lequel le dispositif de commande (8) est en outre configuré pour mettre en pause la première pompe (11), et/ou pour mettre en pause la deuxième pompe (12), et/ou pour commuter la première valve (51) dans la première position de valve fermée, et/ou pour commuter la deuxième valve (52) dans la deuxième position de valve fermée, en réaction audit accéléromètre ayant détecté que le dispositif d'infusion portable est en chute libre et/ou que le dispositif d'infusion portable est secoué au-delà dudit niveau d'agitation de seuil prédéterminé.

5. Dispositif de perfusion portable (1) selon l'une quelconque des revendications précédentes, comprenant en outre :
• un détecteur de bulles de trajet de pompe au réservoir (14) configuré et agencé pour détecter des bulles dans la structure de trajet de fluide de pompe au réservoir (10),
et dans lequel le dispositif de commande (8) est en outre configuré pour :
- en réaction à la détection de bulles par l'au moins un détecteur de bulles à trajet pompe-pompe (14) pendant une première course d'admission du premier piston plongeur (31) dans la première position de valve de communication avec le réservoir de la première valve (51), empêcher la première valve de commuter dans la première position de valve de communication avec le patient pendant ladite première course d'admission ainsi que pendant une première course d'expulsion immédiatement suivante du premier piston plongeur (31), ladite première course d'expulsion immédiatement suivante étant immédiatement à côté de ladite première course d'admission, et
- en réaction à la détection de bulles par l'au moins un détecteur de bulles de trajet de pompe au réservoir (14) pendant une deuxième course d'admission du deuxième piston plongeur (32) dans la deuxième position de valve de communication avec le réservoir de la deuxième valve (52), empêcher la deuxième valve de commuter dans la deuxième position de valve de communication avec le patient pendant ladite deuxième course d'admission ainsi que pendant une deuxième course d'expulsion immédiatement suivante du deuxième piston plongeur (32), ladite deuxième course d'expulsion immédiatement suivante étant immédiatement à côté de ladite deuxième course d'admission.

6. Dispositif de perfusion portable (1) selon la revendication 5, dans lequel le dispositif de commande (8) est en outre configuré pour déterminer une condition d'alerte de bulle de trajet de pompe au réservoir indiquant de détecter de manière répétée des bulles dans la structure de trajet de fluide de pompe au réservoir (10) :
- en réaction à l'apparition d'une détection de bulles par le détecteur de bulles de trajet de pompe au réservoir (14) pendant chacun d'un nombre prédéterminé de cycles immédiatement consécutifs de premières courses d'admission immédiatement consécutives et de premières courses d'expulsion du premier piston plongeur (31) dans la première position de valve de communication avec le réservoir de la première valve (51), et/ou
- en réaction à l'apparition d'une détection de bulles par le détecteur de bulles de trajet de pompe au réservoir (14) pendant chacun d'un nombre prédéterminé de cycles immédiatement consécutifs de deuxièmes courses d'admission immédiatement consécutives et de deuxièmes courses d'expulsion du deuxième piston plongeur (32) dans la deuxième position de valve de communication avec le réservoir de la deuxième valve (52).

7. Dispositif de perfusion portable (1) selon l'une quelconque des revendications précédentes, comprenant en outre :
• un détecteur de bulles de trajet de la pompe au patient (15) configuré et agencé pour détecter des bulles dans la structure de trajet de fluide de la pompe au patient (20);
dans lequel le dispositif de commande (8) est en outre configuré pour déterminer une condition d'alerte de bulle de trajet de la pompe au patient indiquant la détection de bulles dans la structure de trajet de fluide de la pompe au patient (20), en réaction à la détection de bulles par le détecteur de bulles de trajet de la pompe au patient (15) dans la première position de valve de communication avec le patient de la première valve (51), ainsi qu'en réaction à la détection de bulles par le détecteur de bulles de trajet de la pompe au patient (15) dans la deuxième position de valve de communication avec le patient de la deuxième valve (52).

8. Dispositif de perfusion portable (1) selon l'une quelconque des revendications précédentes, comprenant en outre :
• un premier détecteur de bulles de buse de pompe (16) configuré et agencé pour détecter des bulles dans une première buse (91) de la première pompe (11);
dans lequel le dispositif de commande (8) est en outre configuré pour déterminer une première condition d'alerte de bulle de buse de pompe indiquant la détection de bulles dans la première buse (91) de la première pompe (11).

9. Dispositif de perfusion portable (1) selon l'une quelconque des revendications précédentes, comprenant en outre :
• un deuxième détecteur de bulles de buse de pompe (17) configuré et agencé pour détecter des bulles dans une deuxième buse (92) de la deuxième pompe (12);
dans lequel le dispositif de commande (8) est en outre configuré pour déterminer une deuxième condition d'alerte de bulle de buse de pompe indiquant la détection de bulles dans la deuxième buse (92) de la deuxième pompe (12).
